(19) Europäisches Patentamt
European Patent Office
Office européen des brevets

(11) Publication number: **0 132 043 B2**

(12) **NEW EUROPEAN PATENT SPECIFICATION**

(45) Date of publication of the new patent specification: **31.08.94**

(51) Int. Cl.⁵: **C07H 15/04**

(21) Application number: **84303874.6**

(22) Date of filing: **08.06.84**

The file contains technical information submitted after the application was filed and not included in this specification

# (54) Improved process for preparing alkyl glycosides.

(30) Priority: **15.06.83 US 504647**

(43) Date of publication of application:
**23.01.85 Bulletin 85/04**

(45) Publication of the grant of the patent:
**23.12.87 Bulletin 87/52**

(45) Mention of the opposition decision:
**31.08.94 Bulletin 94/35**

(84) Designated Contracting States:
**BE DE FR GB IT NL**

(56) References cited:
**EP-A- 0 092 355**
**EP-A- 0 092 875**
**FR-A- 2 055 595**
**US-A- 2 356 565**
**US-A- 3 547 828**
**US-A- 3 839 318**

**McCutcheon's Detergents & Emulsifiers 1980 North American Edition, p. 199**

**The Organic Chemistry of Sulfur, C.M. Suter, John Wiley & Sons, Inc London 1944, pp. 2 and 75**

**Paul Karrer, "Lehrbuch der Organischen Chemie", 10th Ed. (1948), Georg Theme Verlag, Stuttgart, pp. 122-123**

**L.F.Fieser/M.Fieser "Lehrbuch der Organischen Chemie", 1954, pp.138-139**

**"The Ethyl-sulfuric Acid reaction" by P M Evans and J M.Albertson, 06/01/17, pp. 456-461**

**Journal of Amer. Chem.Society, vol. 56 (1934), pp. 677-679**

(73) Proprietor: **THE PROCTER & GAMBLE COMPANY**
**301 East Sixth Street**
**Cincinnati Ohio 45202 (US)**

(72) Inventor: **Davis, Jerry Edison**
**690 Berkshire Lane**
**Cincinnati Ohio 45220 (US)**
Inventor: **Letton, James Carey**
**11374 Kenshire Drive**
**Forest Park Ohio 45240 (US)**

(74) Representative: **Canonici, Jean-Jacques et al**
**Procter & Gamble European Technical Center N.V.**
**Temselaan 100**
**B-1853 Strombeek-Bever (BE)**



Rank Xerox (UK) Business Services
(3.10/3.09/3.3.3)

## Description

The invention pertains to the preparation of alkyl glycosides by the reaction of a ($C_8$-$C_{30}$) alcohol with a monosaccharide, or other source of monosaccharide moiety in the presence of an acid catalyst.

U.S. Pat. No. 3,839,318, Mansfield, issued October 1, 1974, discloses a process for preparing alkyl glycosides by direct, acid catalyzed reaction of a higher alcohol and a saccharide. The disclosed catalysts are mineral acids such as hydrochloric and sulfuric acid and an acid cation exchange resin.

U.S. Pat No. 3,547,828, issued December 17, 1970, discloses a process for preparing higher alkyl glycosides by first reacting a saccharide with a lower alcohol (e.g. butanol) in the presence of an acid catalyst to form the lower alkyl glycoside, which is then reacted with the higher alcohol too effect transacetylation thereby forming the higher alkyl glycoside. The acid catalysts disclosed are mineral acids (e.g., $H_2SO_4$, $HNO_3$) and organic acids such as p-toluene sulfonic acid and methane sulfonic acid. It is stated that in general any "Lewis acid" may be used. Sulfuric acid is indicated as preferred.

U.S. Pat. No. 3,598,865, Lew, issued August 10, 1971, discloses preparation of higher alkyl glycosides by acid catalyzed reaction of a saccharide and a higher alcohol in the presence of a lower alcohol (designated as a "latent solvent"). The acids disclosed as suitable catalysts are sulfuric, hydrochloric, phosphoric and p-toluenesulfonic acids and boron trifluoride.

It is desirable that alkyl glycosides, particularly those intended for use in consumer products, be essentially colorless, or at least have very low color content. It has been found that color control can be a significant problem when using prior art acid catalysts. It is also desirable that the amount of polysaccharides in surfactant grade alkyl glycosides be kept to a minimum since these do not contribute to detergency. Control of polysaccharide formation can be a significant problem when using the prior art acid catalysts.

It is the object of the present invention to provide an improved acid catalyst for use in preparing alkyl glycosides, thereby achieving improved color of finished product and lower polysaccharide content.

The present invention is directed to an improvement in the acid catalyzed process for preparing alkyl glycosides from saccharides and alcohols, the improvement residing in the use of an acid form of an anionic surfactant as the acid catalyst.

The present invention is a process for preparing alkyl glycosides having from 8 to 30 carbon atoms in the alkyl chain by mixing and reacting a monohydric alcohol containing an alkyl group of from 8 to 30 carbon atoms with a source of monosaccharide moiety and an acid form of an anionic surfactant as catalyst.

The monohydric alcohols containing from 8 to 30 carbon atoms used in the present invention are primary or secondary alcohols, straight or branched chain, saturated or unsaturated, alkyl alcohols. In general, these alcohols have minimal solvent power for the saccharide molecule. Illustrative examples of the monohydric alcohols which may be employed in the present invention are, octyl alcohol, nonyl alcohol, decyl alcohol, dodecyl alcohol, tridecyl alcohol, tetradecyl alcohol, pentadecyl alcohol, hexadecyl alcohol, heptadecyl alcohol, octadecyl alcohol, eicosyl alcohol, pentacosyl alcohol, oleyl alcohol, and 2-methyl-7-ethyl-4-undecanol. A preferred group of alcohols are those having the formula ROH wherein R represents an alkyl radical having from 12 to 18 carbon atoms. The alkyls can be straight or branched chain.

The saccharides used as reactants in the process herein ara monosaccharides which can be alkylated in the "1" position, also sometimes called "reducing monosaccharides." These include hexoses and pentoses. Typical examples of suitable monosaccharides include glucose, manose, galactose, talose, allse, altrose, idose, arabinose, xylose, ribose and lyxose. For reasons of convenience, availability and low cost, the preferred saccharide is glucose.

Instead of monosaccharides, per se, materials which are hydrolyzable to monosaccharides can also be used as reactants in the present process. These include syrups such as corn syrup and molasses. Also the glycosides of the short chain ($C_1$-$C_4$) alcohols, e.g., methyl glycosides and butyl glycosides can be used as reactants which provide the monosaccharide moiety for making glycosides of higher ($C_8$-$C_{30}$) alcohols. The preferred reactants are the monosaccharides, and the process will be described herein primarily in the context of using a monosaccharide, glucose, as the source of saccharide moiety in the reaction.

The amount of alcohol and monosaccharide employed in the process will generally be such that the molar ratio of alcohol to monosacchharide is from 1:1 to 7:1. Preferably the molar ratio is from 1.5:1 to 3:1.

All percentages and ratios set forth herein are "by weight" unless stated otherwise.

The acid catalyst in the process herein is the acid form of an anionic synthetic surfactant.

The most widely known class of anionic synthetic surfactants are organic sulfuric reaction products having in their molecular structure an alkyl group containing from 8 to 22 carbon atoms and a sulfonic acid or sulfuric acid ester group. (Included in the term "alkyl" is the alkyl portion of acyl, alkaryl, alkylpolyethoxy,

alkarylpolyethoxy, alkyl glycerylether and alkyl monoglyceride groups). Examples of anionic synthetic surfactants are alkyl sulfates, especially those obtained by sulfating the higher alcohols ($C_8$-$C_{18}$ carbon atoms) produced by reducing the glycerides of tallow or coconut oil; and alkyl benzene sulfonates, in which the alkyl group contains from 9 to 15 carbon atoms in straight chain or branched chain configuration, e.g., those of the type described in U.S. Pat. Nos. 2,220,099, and 2,477,383.

Other examples of anionic synthetic surfactant compounds useful herein are alkyl glyceryl ether sulfonates, especially those ethers or higher alcohols derived from tallow and coconut oil; coconut oil fatty acid monoglyceride sulfonates and sulfates; alkyl phenol polyalkylenne oxide ether sulfate containing 1 to 10 units of alkylene oxide (e.g., ethylene or propylene) per molecule and wherein the alkyl groups contain 8 to 12 carbon atoms.

Other useful anionic surfactants herein include the 2-acyloxy-alkane-1-sulfonates containing from 2 to 9 carbon atoms in the acyl group and from 9 to 23 carbon atoms in the alkane moiety; alkyl ether sulfates containing from 10 to 20 carbon atoms in the alkyl group and from 1 to 30 moles of ethylene oxide; olefin sulfonates containing from 12 to 24 carbon atoms; and ($\beta$-alkyloxy alkane sulfonates containing from 1 to 3 carbon atoms in the alkyl group and from 8 to 20 carbon atoms in the alkane moiety. Compounds of the foregoing types wherein the sulfate group is replaced by phosphonate are also suitable for use herein.

Many commercially available anionic surfactants are described in *McCutcheon's Detergents and Emulsifiers, North American Edition, MC Publishing co. (1980).*

Anionic surfactants are normally used in the form of their neutralized alkali metal, alkaline earth metal or amine salts. However, when used as catalysts in the process of the present invention, they are used in their unneutralized (i.e., acid) form.

Preferred acid form anionic synthetic surfactants for use herein are:

(a) Alkyl sulfates of the formula

$$RO(C_2H_4O)_nSO_3H$$

wherein R is an alkyl group of from 8 to 22 (preferably 12 to 18) carbon atoms, and n is from 0 to about 6.

(b) Alkylbenzene sulfonates of the formula

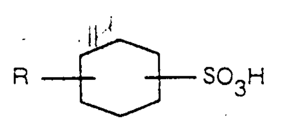

wherein R is alkyl of from 8 to 15, preferably 12 to 14, carbon atoms.

(c) - Alkyl sulfonates of the formula

$$RSO_3H$$

wherein R is alkyl of from 8 to 22, preferably 12 to 18, carbon atoms.

The level of acid surfactant catalyst used in the process of the present invention is generally from 0.0003 to 0.016, preferably from 0.002 to 0.006 moles per mole of monosaccharide.

The process herein is conducted at a temperature in the range of from 100 °C to 140 °C (preferably from 100 °C to 120 °C, and most preferably 105 °C to 112 °C). Water of reaction is removed as the reaction proceeds. This is most conveniently done by distillation.

Typically, the reaction is run by mixing the reactants and catalyst in a reaction vessel equipped with a distillation head for removing water of reaction. The reaction mixture is maintained at a temperature of from 109 °C to 112 °C and the progress of the reaction can be monitored by measurement of the amount of water removed and/or analysis of the unreacted monosaccharide content of the reaction mix. Preferably, the distillation is done under partial vacuum and a flow of nitrogen through the headspace. When the reaction is complete, the reaction mix is neutralized to pH about 6.6 to 7 with a base, e.g., NaOH or $Na_2CO_3$. After neutralization, it is usually desirable to distill the reaction mix to remove substantially all of the excess higher alcohol. This can be done, for example, in a Pope Wiped Film Evaporator, operated at about 160-170 °C and about 66.7 Pa (0.5mmHg) pressure.

A typical procedure for carrying out the process of the invention is as follows. The reactor used is equipped with controlled heat source, vacuum source, nitrogen source, mechanical stirrer and distillation head.

Total Charge to the Reactor

1. Glucose (an amount suitable for the reactor size).
2. Higher alcohol (e.g., $C_{12}$-$C_{14}$) - 2 moles per mole of glucose.
3. Acid $C_{12}$ linear alkylbenzene sulfonate (HLAS) - 0.0056 moles per mole of glucose.
4. Base - to neutralize to pH about 6.7.
5. Solvent - sufficient to make a solution of the base (e.g., $H_2O$ solution of NaOH).

Procedure

1. Glucose and higher alcohol are combined and homogenized. (If glucose monohydrate is used, the homogenized slurry is heated under vacuum to remove the water of hydration.)
2. HLAS is added to the slurry and the temperature raised slowly to about 100°C.
3. The reaction mixture is then heated to 110°C. maintained at that temperature, and stirred at that temperature under a nitrogen atmosphere as water of reaction is removed from the reactor via a distillation head.
4. The reaction is continued until water ceases to be evolved.
5. A solution of base is added to the reaction mix until the pH of a 1% solution of the reaction mixture in water is at 6.7 to 6.8.
6. The neutralized crude product is centrifuged to remove insolubles and then stripped of excess higher alcohol by use of a wiped film evaporator.

Instead of introducing all of the monosaccharide and higher alcohol into the reaction vessel at the same time, the reaction can be conducted by incremental or continuous addition of monosaccharide to the higher alcohol at a controlled rate. This method of conducting the reaction tends to further reduce the production of polysaccharide by-products. When incremental or continuous addition of monosaccharide is used, the rate of addition is preferably controlled such that the amount of unreacted monosaccharidae in the reaction mix does not exceed 5 to 10% of the weight of the reaction mix at any given time, and the overall average amount of unreacted monosaccharide in the reaction mix over the course of the reaction should not exceed about 5% of the weight of the reaction mix. The amount of unreacted monosaccharide in the reaction mix can be monitored by gas chromatography of the trimethylsilyl derivative of the monosaccharide,

The use of acid surfactant catalyst in accordance with the present invention provides several advantages over the use of the most common prior art catalysts for this reaction, such as sulfuric acid and p-toluenesulfonic acid. One advantage is faster reaction rate. This is believed due to the buildup of high concentration of catalyst on the surface of the solid (saccharide) particles which is facilitated by surface active (surfactant) properties of the catalyst. A second advantage is less production of oligo- and polysaccharide by-products. This is believed to be attributable to the faster reaction rate. A third advantage is that the level of colored by-products (and precursors of colored by-products which develop their color in the post-reaction alcohol stripping step) are reduced. This is also believed to be due to the faster reaction rate produced by the catalyst of the invention.

The benefits of the present invention will be illustrated by the following example.

EXAMPLE 1

This example demonstrates the advantages achieved by using a catalyst of the present invention as compared to sulfuric acid and p-toluenesulfonic acid.

I Experimental

(a) Three reactions were run with the acid catalyst as the variable. The catalysts used were: (1) acid $C_{10}$-$C_{14}$ linear alkylbenzene sulfonate (HLAS); (2) p-toluenesulfonic acid (p-TSA); (3) Sulfuric acid.
(b) Equivalent amounts of acid catalyst were used in each reaction, based upon the equivalent weight (sulfuric acid being diprotic while the other catalysts are monoprotic).
(c) Anhydrous glucose was ground and sieved to a uniform particle size (70 mesh) in each instance.
(d) The higher alcohol used was a $C_{12}$-$C_{14}$ blend, all samples taken from the same container and lot.
(e) Reactions were run at atmospheric pressure with a steady flow of nitrogen through the reactor head-space.

*Reaction Conditions – Description*

| Chemicals used: | |
|---|---|
| Glucose | 100 g (0.55 mole) |
| Alcohol | 214 g (1.1 moles) |
| Acid | 0.00315 mole (0.0015 mole for $H_2SO_4$) |
| Base | 0.00315 mole |

Procedure:

A one liter flask equipped with a distillation condenser, overhead stirrer, nitrogen inlet and thermometer was used. The reaction flask was heated by heating mantle with the reaction temperature controlled by a thermo-watch.

The higher alcohol and acid catalyst were combined in the reaction flask and the temperature raised to 85°C while stirring under a nitrogen atmosphere. At 85°C, 100 grams of glucose were added and the reaction timed from that point.

The temperature was raised to 110°C and the reaction continued with stirring under a nitrogen atmosphere for three hours. The temperature was held at 109-112°C over the total reaction time.

Heating was discontinued at the end of the three hour period and the mixture cooled to 90°C before neutralization with 0.00315 mole of sodium ethoxide.

Samples were taken at various intervals to determine the amount of residual glucose in the mixture.

Data

1. *Reaction Rates*

The data presented in Table 1 show the % residual glucose (% remaining of the initial amount added). This remaining glucose was determined by settling of the glucose from the reaction mix and measuring volume of residual glucose relative to sample volume. Sample taken immediately after addition of glucose to the reactor is set at 100%.

TABLE 1

| Time (Hrs.) | % Residual Glucose | | |
|---|---|---|---|
| | HLAS | p-TSA | $H_2SO_4$ |
| 0 | 100 | 100 | 100 |
| ¼ | 77 | 81.3 | 84.5 |
| ½ | 65 | 71.3 | 72.4 |
| 1 | 50 | 64.5 | 55.5 |
| 1½ | 37.5 | 61.6 | -* |
| 2 | 25 | -* | -* |

*Samples too viscous and foamy to measure.

These data show a faster consumption of glucose, and therefore a faster reaction rate in the HLAS catalyzed reaction. Also, the higher fluidity of the HLAS reaction produced better reaction control and mass transfer.

2. *Composition (After Alcohol Removal)*

The composition data in Table 2 were obtained on the finished reaction product after neutralization and stripping of excess higher alcohol. The data show the average number of glucoside units (n) in the alkyl glucoside, and the percent glucose and polysaccharides in the reaction product. Alkyl glucoside and

glycose analyses were made by gas chromatography while polysaccharides were measured by HPLC.

TABLE 2

| | HLAS | p-TSA | $H_2SO_4$ |
|---|---|---|---|
| Distribution of of $RG_n$(n) | 1.61 | 1.61 | 1.6 |
| % Glucose | 0.2 | 0.1 | 0.1 |
| % Polysaccharides | 9.2 | 11.3 | 24.1 |

These data show a higher level of polysaccharide (an undesirable by-product) in the products made with p-TSA and $H_2SO_4$ catalyst.

3. *Color – Color Precursors*

The development of undesirable color in acid catalyzed glucose reactions may be monitored by U.V.-VIS spectrophotometry. Known color precursors, visible in the ultraviolet region form initially, followed by color bodies which absorb in the visible region of the spectrum.

This data is presented as absorbence, which is linear in relationship to concentration. Absorbence was measured on 1% solutions (in 70/30 ethanol/water) of samples of reaction mix (after neutralization, and stripping of excess $C_{12}$-$C_{14}$ alcohol by distillation at 130°C and about 667 Pa (5 mm Hg) for about 2 hours). Known color precursors such as furan derivatives and other unsaturated carbonyl compounds, absorb in the range of 270-350 nm. Color bodies absorb at 40-500 nm. and above, specifically at 440 and 470 nm. The absorbence data are shown in Table 3.

TABLE 3

| nm. | Absorbence | | |
|---|---|---|---|
| | HLAS | p-TSA | $H_2SO_4$ |
| 270 | 0.922 | 1.663 | 1.6429 |
| 300 | 0.690 | 0.8700 | 1.3798 |
| 330 | 0.5209 | 0.7040 | 1.1348 |
| 350 | 0.4521 | 0.6350 | 1.0232 |
| 440 | 0.2585 | 0.4005 | 0.6708 |
| 470 | 0.2252 | 0.3585 | 0.6066 |

These data show that the HLAS-catalyzed reaction produced lower levels of color precursors and better color in finished product.

**Claims**

**1.** A process for preparing alkyl glycosides having from 8 to 30 carbon atoms in the alkyl chain by mixing and reacting a monohydric alcohol containing an alkyl group of from 8 to 30 carbon atoms with a source of monosaccharide moiety and an acid form of an anionic surfactant as catalyst.

**2.** A process according to claim 1 wherein the acid form of the anionic surfactant catalyst is an organic sulfuric reaction product having in its molecular structure an alkyl moiety containing from 8 to 22 carbon atoms and an acid moiety selected from sulfonic acid and sulfuric acid ester.

**3.** A process according to claim 1 or claim 2 wherein the source of monosaccharide moiety is a monosaccharide.

**4.** A process according to any of claims 1 to 3 wherein the source of monosaccharide moiety is glucose.

**5.** A process according to any of claims 1 to 4 wherein the acid form of the anionic surfactant catalyst is selected from:

(a) alkyl sulfates of the formula

$RO\ (C_2H_4O)_nSO_3H$

wherein R is an alkyl group of from 8 to 22 carbon atoms, and n is from 0 to 6,
(b) alkylbenzene sulfonates of the formula

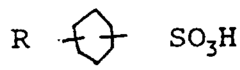

wherein R is alkyl of from 8 to 15 carbon atoms, and
(c) alkyl sulfonates of the formula $RSO_3H$ wherein R is an alkyl of from 8 to 22 carbon atoms.

**6.** A process according to any of claims 1 to 5 wherein the acid form of the anionic surfactant catalyst is an alkylbenzene sulfonate of the formula

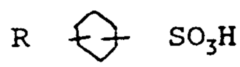

wherein R is an alkyl of from 12 to 14 carbon atoms.

**7.** A process according to any of claims 1 to 6 wherein the alkyl group of the alcohol contains 12 to 18 carbon atoms.

## Patentansprüche

**1.** Verfahren zur Herstellung von Alkyl-Glycosiden mit einer Alkylkette mit 8 bis 30 Kohlenstoffatomen durch Mischen und Umsetzen eines einwertigen Alkohols mit 8 bis 30 Kohlenstoffatomen in der Alkylkette mit einer den Monosaccharidteil liefernden Verbindung und einem anionischen Tensid in saurer Form als Katalysator.

**2.** Verfahren gemäß Anspruch 1, worin der in saurer Form vorliegende anionische oberflächenaktive Katalysator ein organisches Sulfurierungsreaktionsprodukt ist, welches in seiner Molekularstruktur einen Alkylrest mit 8-22 Kohlenstoffatomen hat und einen Säurerest aufweist, welcher ausgewählt ist aus Sulfonsäure und Schwefelsäureester.

**3.** Verfahren gemäß Anspruch 1 oder 2, worin die Monosaccharidquelle ein Monosaccharid ist.

**4.** Verfahren gemäß einem der Ansprüche 1-3, worin die Monosaccharidquelle Glucose ist.

**5.** Verfahren gemäß einem der Ansprüche 1 bis 4, worin der in saurer Form vorliegende anionische oberflächenaktive Katalysator ausgewählt wird aus:
a) Alkylsulfaten der Formel

$RO(C_2H_4O)_nSO_3H$

worin R eine Alkylgruppe mit 8-22 Kohlenstoffatomen und n gleich 0 bis 6 bedeutet,
b) Alkylbenzolsulfonaten der Formel

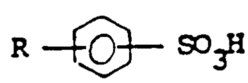

worin R einen Alkylrest mit 8 bis 15 Kohlenstoffatomen bedeutet, und
c) Alkylsulfonaten der Formel

$RSO_3H$

worin R eine Alkylgruppe mit 8 bis 22 Kohlenstoffatomen bedeutet.

**6.** Verfahren gemäß einem der Ansprüche 1 bis 5, worin der in saurer Form vorliegende anionische oberflächenaktive Katalysator ein Alkylbenzolsulfonat der Formel

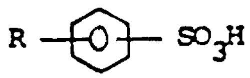

ist, worin R einen Alkylrest mit 12 bis 14 Kohlenstofatomen bedeutet.

**7.** Verfahren gemäß einem der Ansprüche 1 bis 6, worin die Alkylgruppe des Alkohols 12 bis 18 Kohlenstoffatome enthält.

## Revendications

**1.** Procédé de préparation d'alkylglycosides comportant de 8 à 30 atomes de carbone dans la chaîne alkyle, qui consiste à mélanger et à faire réagir un monoalcool contenant un groupe alkyle de 8 à 30 atomes de carbone avec une source de groupement monosaccharide et une forme acide d'un tensioactif anionique.

**2.** Procédé selon la revendication 1 dans lequel la forme acide du catalyseur tensioactif anionique est un produit organique de réaction sulfurique comportant, dans sa structure moléculaire, un groupement alkyle contenant de 8 à 22 atomes de carbone et un groupement acide choisi parmi un ester d'acide sulfonique et un ester d'acide sulfurique.

**3.** Procédé selon la revendication 1 ou 2, dans lequel la source de groupement monosaccharide est un monosaccharide.

**4.** Procédé selon l'une quelconque des revendications 1 à 3, dans lequel la source de groupement monosaccharide est le glucose.

**5.** Procédé selon l'une quelconque des revendications 1 à 4, dans lequel la forme acide du catalyseur tensioactif anionique est choisie parmi :
(a) les alkylsulfates de formule

$RO(C_2H_4O)_nSO_3H$

dans laquelle R est un groupe alkyle de 8 à 22 atomes de carbone et n est de 0 à 6,
(b) les alkylbenzène-sulfonates de formule

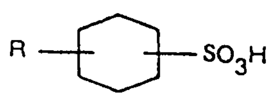

dans laquelle R est un groupe alkyle de 8 à 15 atomes de carbone, et
(c) les alkylsulfonates de formule

$RSO_3H$

dans laquelle R est un groupe alkyle de 8 à 22 atomes de carbone.

**6.** Procédé selon l'une quelconque des revendications 1 à 5, dans lequel la forme acide du catalyseur tensioactif anionique est un alkylbenzène-sulfonate de formule

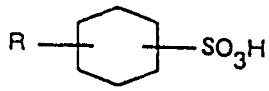

dans laquelle R est un groupe alkyle de 12 à 14 atomes de carbone.

7. Procédé selon l'une quelconque des revendications 1 à 6, dans lequel le groupe alkyle de l'alcool contient de 12 à 18 atomes de carbone.